# EUROPEAN PATENT APPLICATION

(11) **EP 4 368 707 A1**
(43) Date of publication of application: **15.05.2024**
(21) Application number: 22837864.2
(22) Date of filing: 24.06.2022
(51) Int. Cl.: C12N 7/00, C12N 15/10

(54) **METHOD FOR PRODUCING REASSORTANT REOVIRIDAE VIRUS, AND VECTOR LIBRARY FOR SAME**

(30) Priority: 06.07.2021 KR 20210088575
(71) Applicant: SK Bioscience Co., Ltd., Bundang-gu, Seongnam-si, Gyeonggi-do, 13494 (KR)
(72) Inventor: SEO, Ki Weon, Seongnam-si, Gyeonggi-do 13494 (KR); KWON, Tae Woo, Seongnam-si, Gyeonggi-do 13494 (KR); JUNG, Seo Yeon, Seongnam-si, Gyeonggi-do 13494 (KR); PARK, Min Jung, Seongnam-si, Gyeonggi-do 13494 (KR); LEE, Kun Se, Seongnam-si, Gyeonggi-do 13494 (KR); LEE, Hyun Joo, Seongnam-si, Gyeonggi-do 13494 (KR)
(74) Representative: Weickmann & Weickmann PartmbB
(86) International application number: PCT/KR2022/009016
(87) International publication number: WO 2023/282510

(57) **Abstract**

The present application relates to a method for producing a reassortant *Reoviridae* virus and a vector library for the same, and provides: a method for producing a reassortant *Reoviridae* virus by using a cell line into which an RNA polymerase is introduced and an expression vector library according to an aspect; a reassortant *Reoviridae* virus produced by the method; and an expression vector library for producing reassortant rotavirus.

## Description

### Technical Field

The present disclosure relates to a method for producing a reassortant *Reoviridae* virus and a vector library for the same. This application is based on and claims priority to Korean Patent Application No. 10-2021-0088575, filed on July 6, 2021, in the Korean Intellectual Property Office, the disclosure of which is incorporated by reference herein in its entirety.

### Background Art

*Reoviridae* viruses are a family of viruses having genomes consisting of double-stranded RNA. These viruses have genome segments that are packaged in a multilayered capsid, and have a polyhedral shape with a size of about 70 nm to about 85 nm without having an envelope containing lipids. The *Reoviridae* may be classified into two subfamilies, *Sedoreovirinae* and *Spinareovirinae*, and infections with such viruses mainly affect the digestive or respiratory system. Human rotavirus (HRV) is a major virus that causes diarrhea in infants, and in Korea and around the world, 95 % of children under 5 years old have been infected with HRV at least once, and HRV is known to account for about 40 % of diarrhea patients every year worldwide. HRV infection is apparent infection with rotavirus, and the main route of transmission of HRV infection is through fecal-oral transmission and the incubation period of HRV infection is about 24 hours to about 72 hours. HRV infection is a disease that can cause dehydration by vomiting, fever, and non-bloody watery diarrhea. It mainly occurs in infants and children, but occasionally mass outbreaks occur in geriatric wards.

The rotavirus has genomes consisting of 11 segments of dsRNA, and is within a non-enveloped icosahedral capsid with a diameter of about 75 nm, wherein the capsid is a three-layered protein capsid consisting of an outer capsid, an inner capsid, and a core protein. Each segment encodes one of 6 structural proteins (VP1, VP2, VP3, VP4, VP6, and VP7) and 5 non-structural proteins. The rotavirus may be classified into 8 types (groups A to H), wherein infection with the group A rotavirus, the group B rotavirus, and the group C rotavirus, especially with the group A rotavirus, is common in humans. The group A rotavirus may be classified into P or G serotypes depending on VP4 and VP7 proteins. In detail, a protease-sensitive protein, VP4, determines P serotypes, and a glycoprotein, VP7, determines G serotypes. The rotavirus serotype may be determined according to combinations of these proteins. Genes for determining the serotypes are transferred separately to progeny viruses, and thus various forms of combinations may occur. In particular, each of the rotavirus serotypes in various forms of combinations may have the characteristic of not providing cross-protection.

Due to the rotavirus characteristic of not providing cross-protection across the serotypes, there has been a need for the development of effective rotavirus vaccines that can protect against infection with each serotype. To date, live-attenuated oral vaccines and animal-human recombinant vaccines have been used, but failed to exhibit sufficient protective ability against infections with different serotypes. Rotashield, developed by Wyeth-Ayerst Laboratories in the United States, is a tetravalent live-attenuated vaccine combining the most prevalent G serotypes (G1 to G4), and was approved by the FDA and utilized as basic vaccination. However, due to intussusception cases, use of the Rotashield vaccine has been discontinued. Accordingly, interest in a naturally/artificially generated/manufactured reassortant rotavirus (including virus-like particles) as a material for vaccines against rotavirus infection is increasing.

The reassortant rotavirus is a virus produced by combining RNA segments of different strains when a single cell is simultaneously infected with several strains of virus. It may be produced spontaneously in nature, but the probability of such spontaneous occurrence is very low. Even if reassortment does occur, the resulting virus will be very severely attenuated compared to a wild-type virus, and thus will be difficult to isolate. In addition, the reassortant rotavirus may be produced by simultaneously infecting an artificially cultured single cell with both a bovine rotavirus and a human rotavirus. However, it has disadvantages that the resulting virus is produced in much smaller quantities compared to a wild-type virus and is attenuated, and thus, unless a selective pressure is applied, the rate gradually decreases as a passage progresses, making it very difficult to isolate and recover the desired reassortant rotavirus. To solve the problems above, a process of screening the reassortant rotavirus by using neutralizing antibodies specific to proteins of a gene to be replaced has been additionally introduced, but there are technical limitations in that securing monoclonal neutralizing antibodies with the aforementioned functionality should be addressed first and additional screening processes are required for each of all serotypes.

Under this technical background, as part of the development of vaccines against the *Reoviridae* viruses including rotavirus, diverse studies are being conducted to efficiently produce reassortant viruses (Korean Patent Publication No. 10-2019-0108882), but the situation is still inadequate.

### Disclosure

### Technical Problem

An aspect provides a method for producing a reassortant *Reoviridae* virus by using a cell line into which a RNA polymerase gene is introduced and an expression vector library for rotavirus.

Another aspect provides a reassortant *Reoviridae* virus produced by the method and an immunogenic composition including the same.

Another aspect provides an expression vector library for producing reassortant rotavirus.

Other purposes and advantages of the present disclosure will become more obvious with the following detailed description, claims, and drawing. Contents not described herein will be sufficiently recognized and inferred by those skilled in the technical field of the present application or in a similar technical field therewith, and thus descriptions of such contents will be omitted.

### Technical Solution

An aspect provides a method for producing a reassortant *Reoviridae* virus, the method including: transfecting a first cell line, into which an RNA polymerase gene is introduced, into an expression vector library including a foreign gene; culturing a first culture by culturing, in a growth medium, the first cell line undergoing the transfection; culturing a second culture by adding a second cell line to the first culture that has been cultured; and culturing a third culture by adding trypsin to the second culture that has been cultured, wherein the expression vector library includes a gene segment of cDNA of the *Reoviridae* virus and a promoter capable of binding to the RNA polymerase.

As used herein, the term "*Reoviridae* virus" is a family of viruses having genomes consisting of double-stranded RNA. This virus has genome segments that are packaged in a multilayered capsid, and have a polyhedral shape with a size of about 70 nm to about 85 nm without having an envelope containing lipids. The *Reoviridae* virus may be, for example, one selected from the group consisting of the genera *Rotavirus*, *Cardoreovirus*, *Mimoreovirus*, *Orbivirus*, *Phytoreovirus*, *Seadornavirus*, *Aquareovirus*, *Coltivirus*, *Cypovirus*, *Dinovernavirus*, *Fijivirus*, *Idnoreovirus*, *Mycoreovirus*, *Orthoreovirus*, and *Oryzavirus.* The *Reoviridae* virus may be, for example, a virus of the genus *Rotavirus*, *Reovirus*, *Orbivirus*, or *Coltivirus*, and may be, for example, rotavirus.

The *Reoviridae* virus may proliferate in the cytosol and may be released by cell destruction, causing infection in various hosts such as humans, fish, insects, plants, etc., and among the aforementioned viruses, a virus of the genus *Rotavirus*, *Reovirus*, *Obivirus*, or *Coltivirus* is known to affect humans. Accordingly, diverse studies are being conducted to produce vaccine compositions, specifically live vaccines, to prevent infection with the *Reoviridae* viruses. However, due to the genetic characteristics that the double-stranded RNA genomes are divided into multiple segments and the existence of various serotypes, there are difficulties in producing vaccines. Meanwhile, the conventional vaccine manufacturing methods of delivering each segment of synthesized viral RNA directly to cells are difficult to apply to the preparation of the *Reoviridae* virus consisting of multiple gene segments, due to the instability of RNA itself and the subsequent difficulties in the intracellular delivery. In addition, the vaccine manufacturing methods using plasmid DNA utilize the transcription of a host cell and RNA modification mechanisms, but have problems of reduced delivery efficiency due to additional transport into the cell nucleus and reduced transcriptional efficiency due to competition reactions among promoters under the presence of a limited amount of polymerases in a host cell. Under this technical background, the present disclosure is intended to solve the problems in the prior art, and confirms that the reassortant *Reoviridae* virus can be obtained/produced by using a cell line stably expressing a T7 RNA polymerase and an expression vector library according to an aspect, thereby completing the present disclosure.

A method for producing the reassortant *Reoviridae* virus may be described in detail in each step as follows.

First, the method may include transfecting a first cell line, into which an RNA polymerase gene is introduced, into an expression vector library including a foreign gene.

As used herein, the term "RNA polymerase" refers to an enzyme that synthesizes primary transcript RNA from DNA. The RNA polymerase may be, for example, a T7 RNA polymerase, a T3 RNA polymerase, an SP6 RNA polymerase, or a mitochondrial polymerase (POLRMT), specifically, an RNA polymerase that acts in the cytosol, and more specifically, a T7 RNA polymerase. The T7 RNA polymerase is a gene product of bacteriophage T7. Unlike other RNA polymerases, the T7 RNA polymerase acts in the cytosol rather than in the nucleus, and thus an additional import process into the cell nucleus is not required. In addition, the T7 RNA polymerase exhibits excellent transcription efficiency, enabling simultaneous transcription of cDNA plasmids of each of the *Reoviridae* viruses consisting of a plurality of gene segments.

As used herein, the term "expression vector library" refers to a collection of independent expression vectors for a plurality of gene segments of the *Reoviridae* virus. For example, when the expression vector library is for the expression of rotavirus, the expression vector library may be for the expression of gene segments of VP1, VP2, VP3, VP4, VP6, VP7, NSP1, NSP2, NSP3, NSP4, and NSP5. For example, the expression vector library for rotavirus may refer to a combination of a total of 11 expression vectors for VP1, VP2, VP3, VP4, VP6, VP7, NSP1, NSP2, NSP3, NSP4, and NSP5, each being selected for each gene segment, and each expression vector from the expression vector library may include a promoter capable of binding to RNA polymerase and a gene segment of rotavirus cDNA.

As used herein, the term "transfection" refers to the introduction of a purified virus nucleic acid or plasmid into an eukaryotic cell. For example, the transfection may include insertion/introduction of genes of a RNA polymerase, e.g., a T7 RNA polymerase, into a cell, or insertion/introduction of a gene segment of the *Reoviridae* virus into a cell.

In an embodiment, the first cell line is a cell line into which an RNA polymerase and a foreign gene have been genetically introduced, and may be BHK21 cell line, HEK293 cell line, HeLa cell line, CHO cell line, LNCaP cell line, A549 cell line, or hepG2 cell line, and for example, may be BHK21 cell line expressing an RNA polymerase. Here, the term "foreign gene" refers to a gene of the *Reoviridae* virus to be reassorted, and specifically, to a gene segment of cDNA of the *Reoviridae* virus. Each gene segment of cDNAs may be isolated/obtained from the same or heterogeneous individuals. For example, the first cell line may be prepared in a way that an expression vector including a constitutive promoter and a T7 RNA polymerase gene operably linked thereto is introduced into BHK21 cell line, and then a high-expression cell line is screened therefrom. However, embodiments are not limited thereto.

In an embodiment, the total DNA weight of the expression vector library may be 1 ug to 20 ug per 1×10⁴ cells to 1×10⁶ cells, for example, 1 ug to 17 ug, 1 ug to 14 ug, 1 ug to 11 ug, 1 ug to 8 ug, 1 ug to 5 ug, 1 ug to 2 ug, 5 ug to 20 ug, 5 ug to 17 ug, 5 ug to 14 ug, 5 ug to 11 ug, 5 ug to 8 ug, 10 ug to 20 ug, 10 ug to 17 ug, 10 ug to 14 ug, or 10 ug to 11 ug. When the total DNA weight is too low or high beyond the ranges above, the yield of a desired reassortant virus may be significantly reduced or may not be obtained at all, due to the genetic characteristics of the *Reoviridae* virus.

In an embodiment, the expression vector library may include a promoter capable of binding to the RNA polymerase, and examples of the promoter may be a T7 promoter, an Sp6 promoter, or a CMV promoter.

In an embodiment, the expression vector library may further include an expression vector for D1R or D12L encoding a capping enzyme. For example, since RNA transcribed by the T7 RNA polymerase in the cytosol has not undergone capping or polyadenylation, the expression vector may be selectively added to the expression vector library for the *Reoviridae* virus to improve stability of the transcribed RNA. However, in the case of rotavirus, due to the presence of VP3, the expression vector may be selectively included, and similarly, expression vectors for D1R or D12L may be selectively included for other *Reoviridae* viruses. Here, the molar ratio of the expression vector for D1R or D12L to the expression vector for the gene segments of the *Reoviridae* virus may be 1:4 to 1:6, and for example, may be 1:4 to 1:5.5, 1:4 to 1:5.0, 1:4 to 1:4.5, 1:4.5 to 1:6.0, 1:4.5 to 1:5.5, 1:4.5 to 1:5.0, 1:5.0 to 1:6.0, or 1:5.0 to 1:5.5. However, the molar ratio is not limited thereto as long as it is within a range that can maintain the stability of the transcribed RNA.

Afterwards, the method may include culturing a first culture by culturing the transfected first cell line in a growth medium.

In an embodiment, the growth medium may be Dulbeco's modified Eagle's medium (DMEM), Eagle's minimum essential medium (EMEM) ,or Glasgow minimum essential medium (GMEM), wherein the growth medium may be selectively supplemented with fetal bovine serum (FBS). When a growth medium supplemented with FBS is used, in a subsequent step, the method may further include replacing the growth medium with an FBS-free medium.

In an embodiment, the replacing of the growth medium may be performed prior to a subsequent step of culturing a third culture, and may be, for example, performed 20 hours after the transfection. In detail, it may be performed at any point between 20 hours to 24 hours after the transfection, at any point between 24 hours to 28 hours after the transfection; or at any point between 28 hours to 32 hours after the transfection.

The culturing of the first culture may be performed under conditions of 30 °C to 38 °C, for example, 30 °C to 36 °C, 30 °C to 34 °C, 30 °C to 32 °C, 32 °C to 38 °C, 32 °C to 36 °C, 32 °C to 34 °C, 34 °C to 38 °C, 34 °C to 36 °C, or 36 °C to 38 °C, for 1 hour to 4 hours, for example, 1 hour to 3 hours, 1 hour to 2 hours, 2 hours to 4 hours, 2 hours to 3 hours, or 3 hours to 4 hours.

Afterwards, the method may include culturing a second culture by adding a second cell line to the cultured first culture.

As used herein, the term "first culture" refers to a product obtained through the culturing of the transfected first cell line in the growth medium, and the first culture may include the transfected cell line and growth medium components.

As used herein, the term "second culture" refers to a substance obtained by adding a second cell line to the first culture, which is the product obtained through the culturing of the transfected first cell line in the growth medium, and the second culture may include the transfected cell line, growth medium components, and the second cell line.

In an embodiment, the second cell line is for promoting proliferation/amplification of the prepared reassortant *Reoviridae* virus, and examples thereof may include MA104 cell line, Vero cell line, HEK cell line, human intestinal epithelial cells, HT-29 cell line, Caco2 cell line, LLC-MK2 cell line, FRhL-2 cell line, CV-1 cell line, COS-7 cell line, BSC-1 cell line, or BGM cell line. For example, the second cell line may be MA104 cell line, which is a monkey kidney cell line.

The culturing of the second culture is to induce amplification of the reassortant *Reoviridae* virus produced from the transfected cells. When culturing the transfected cell line in combination with the second cell line having excellent sensitivity and replication ability to the *Reoviridae* virus, the reassortant *Reoviridae* virus initially produced in a small quantity may be amplified. Here, the second cell line may be added at a concentration of 1×10² cells to 3×10⁶ cells/well, for example, 1×10² cells to 3×10⁵ cells/well, 1×10² cells to 3×10⁴ cells/well, 1×10² cells to 3×10³ cells/well, 1×10² cells to 3×10² cells/well, 3×10² cells to 3×10⁶ cells/well, 3×10² cells to 3×10⁵ cells/well, 3×10² cells to 3×10⁴ cells/well, or 3×10² cells to 3×10³ cells/well.

In an embodiment, in the culturing of the second culture, the second cell line may be added to the first culture 6 hours to 12 hours after the transfection, and for example, may be added at any point between 6 hours to 8 hours after the transfection, at any point between 8 hours to 10 hours after the transfection, or at any point between 10 hours to 12 hours of the transfection, at any point between 12 hours to 14 hours after the transfect, or at any point between 14 hours to 18 hours after the transfection. When the addition time is too early or too late beyond the ranges above, the yield of a desired reassortant virus may be significantly reduced or may not be obtained at all due to the genetic characteristics of the *Reoviridae* virus.

The culturing of the second culture may be performed under conditions of 30 °C to 38 °C, for example, 30 °C to 36 °C, 30 °C to 34 °C, 30 °C to 32 °C, 32 °C to 38 °C, 32 °C to 36 °C, 32 °C to 34 °C, 34 °C to 38 °C, 34 °C to 36 °C, or 36 °C to 38 °C, for 24 hours to 36 hours, for example, 24 hours to 32 hours, 24 hours to 28 hours, 24 hours to 36 hours, 28 hours to 33 hours, or 33 hours to 36 hours.

Afterwards, the method may include culturing a third culture by adding trypsin to the cultured second culture.

As used herein, the term "third culture" refers to a substance obtained by adding trypsin to a product obtained through the culturing of the second culture, and the third culture may include the transfected first cell line, the second cell line, components of the FBS-free medium, and an appropriate amount of trypsin.

The culturing of the third culture is for inducing reinfection with the reassortant *Reoviridae* virus. In this regard, when trypsin is to the aforementioned medium and cultured, the reassortant *Reoviridae* virus in a small quantity may be reinfected with the second cell line. Here, the trypsin may be added at a concentration of 0.1 ug/ml to 1 ug/ml, for example, 0.1 ug/ml to 0.8 ug/ml, 0.1 ug/ml to 0.6 ug/ml, 0.1 ug/ml to 0.4 ug/ml, 0.1 ug/ml to 0.2 ug/ml, 0.3 ug/ml to 1 ug/ml, 0.3 ug/ml to 0.8 ug/ml, 0.3 ug/ml to 0.6 ug/ml, or 0.3 ug/ml to 0.4 ug/ml.

In an embodiment, in the culturing of the third culture, the trypsin may be added to the second culture 36 hours to 48 hours after the transfection, for example, at any point between 36 hours to 48 hours after the transfection, at any point between 36 hours to 42 hours after the transfection, at any point between 42 hours to 48 hours after the transfection, or at any point between 48 hours to 54 hours after the transfection. When the addition time is too early beyond the ranges above, there is a risk that the cell line may be isolated early from the plate, and when the addition time is too late beyond the ranges above, the yield of a desired reassortment virus may be reduced or may not be obtained at all due to the genetic characteristics of the *Reoviridae* virus.

The culturing of the third culture may be performed for 48 hours to 84 hours, for example, 48 hours to 72 hours, 48 hours to 60 hours, 60 hours to 84 hours, 60 hours to 72 hours, or 72 hours to 84 hours. The method may further include obtaining a reassortant *Reoviridae* virus by a known method from the culture cultured as described above.

According to the method according to an aspect, the reassortant *Reoviridae* virus produced through a series of methods described above may selectively have any one serotype or a combination of serotypes, depending on the expression vector library, and an effective amount of the reassortant *Reoviridae* virus may be prepared without an additional screening process.

Another aspect provides the reassortant *Reoviridae* virus prepared by the aforementioned method and an immunogenic composition including the same.

Among the terms or elements mentioned in the following reassortant *Reoviridae* virus and immunogenic composition, those already mentioned in the description of the preparation method above are the same as described above.

As used herein, the term "immunogenicity" refers to the ability of a composition to induce an immune response against a specific pathogen, wherein the immune response may be a cellular immune response mediated primarily by cytotoxic T-cells and cytokine-generating T-cells or a humoral immune response mediated primarily by helper T-cells and then activating B-cells to produce antibodies.

The immunogenic composition may include at least one carrier, a pharmaceutically acceptable stabilizer, and a modified live virus formulated with an adjuvant. A carrier suitable for use may include saline, phosphate buffered saline, potassium dihydrogen phosphate, dipotassium hydrogen phosphate, monosodium glutamate, minimal essential medium (MEM), or a buffer for MEM and HEPES. The stabilizer contributes to the preservation of viability and infectivity of viruses during long-term storage of lyophilized products at refrigerated temperatures and room temperature, especially during a lyophilization process, and for a period sufficient to enable short-term transport from the cold chain. For example, the immunogenic composition may further include a protein stabilizer selected from human serum albumin and/or collagen, hydrolyzed collagen or gelatin, and hydrolyzed gelatin, or a sugar stabilizer selected from sucrose, mannitol, sorbitol, trehalose, and dextran. In addition, the immunogenic composition may include an adjuvant that enhances the immunogenicity against viruses and induces protective immunity by single administration, and may further include a bulking agent selected from the group consisting of lactose, sucrose, mannitol, trehalose, and the like. In addition, the immunogenic composition may further include known ingredients associated with the formulation of live virus vaccines.

The immunogenic composition may be in any form known in the art, and for example, may be in the form of liquids and injections, but embodiments are not limited thereto. For liquids or injections, 10 % to 40% of propylene glycol and sodium chloride in an amount sufficient to prevent haemolysis (e.g., about 1%) may be included. For liquids or injections, any diluent or buffer known in the art may be included. In addition, the immunogenic composition may be prepared immediately before use by preserving the reassortant *Reoviridae* virus in a container, such as a vial, and adding the necessary carrier, adjuvant, saline solution, etc. to injections before use.

Another aspect provides an expression vector library for the preparation of reassortant rotavirus, including a plurality of expression vectors, each including a T7 promoter capable of binding to a T7 RNA polymerase and gene segments of cDNA of rotavirus operably linked to the T7 promoter, wherein each of the plurality of expression vectors may include gene segments of cDNA of any one of VP1, VP2, VP3, VP4, VP6, VP7, NSP1, NSP2, NSP3, NSP4, and NSP5.

Among the terms or elements mentioned in the following expression vector library for the preparation of reassortant rotavirus, those already mentioned in the description of the preparation method above are the same as described above.

As used herein, the term "reassortant rotavirus" refers to a virus formed by combining the 11 dsRNA segments constituting rotavirus from two or more origins, and may be used interchangeably with the term, reassortant rotavirus or artificial recombinant rotavirus. The dsRNA segment of the reassortant rotavirus consists of six structural proteins (VP1, VP2, VP3, VP4, VP6, and VP7) and five non-structural proteins (NSP1, NSP2, NSP3, NSP4, and NSP5). In detail, it is known that VP1 is an RNA polymerase present within the core of viral particles and synthesizes mRNA used for replication of RNA segments, VP2 forms the core of viral particles, and VP3 is a guanylate transferase that contributes to mRNA stabilization by catalyzing the addition of the 5' cap resulting from post-transcriptional modification of mRNA. In addition, VP4, as a capsid protein present on a virus surface, is involved in cell invasion of a virus, and plays a role in determining a P serotype. In addition, VP6, as a main component of a capsid, exhibits high antigenicity, and VP7, as a capsid protein present on a virus surface, has a role in determining a G serotype.

As used herein, the term "vector" refers to a genetic construct that allows expression of a target protein in a suitable host cell, and also refers to a genetic construct including regulatory elements operably linked to express a gene insert. A vector according to an embodiment may include expression regulatory factors, such as a promoter, an operator, a start codon, a stop codon, a polyadenylation signal, and/or an enhancer, and the promoter of the vector may be constitutive or inducible. In addition, the vector may be an expression vector capable of stably expressing a target protein in a host cell. For the expression vector, a conventional vector in the art used to express a foreign protein in plants, animals, or microorganisms may be used. The recombinant vector may be constructed through various methods known in the art. For example, the vector may include a selectable marker for selecting a host cell including a vector, and in the case of a replicable vector, it may include an origin of replication. In addition, the vector may be self-replicated or introduced into host DNA, wherein the vector may be selected from the group consisting of a plasmid, lentivirus, adenovirus, adeno-associated virus, retrovirus, herpes simplex virus, and vaccinia virus.

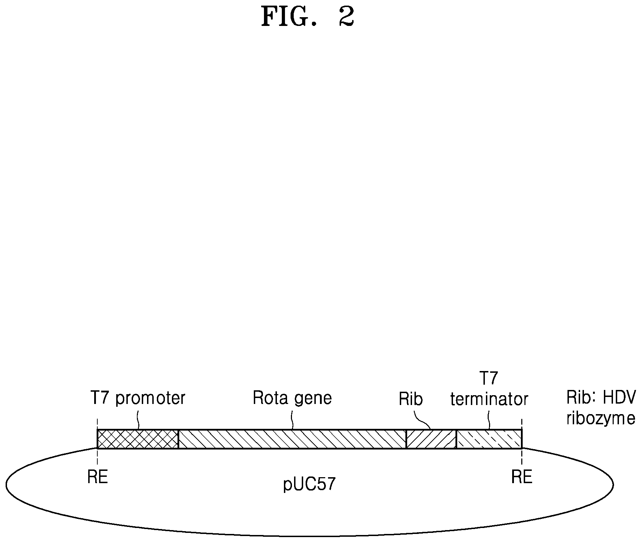

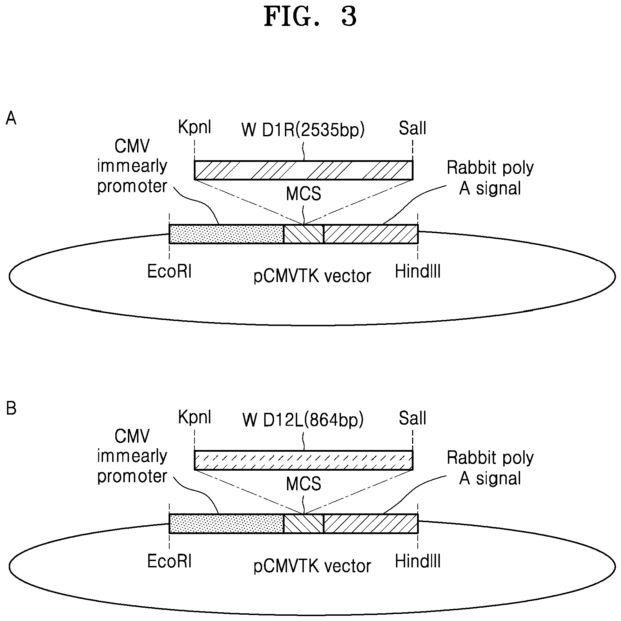

The vector may include a promoter capable of binding to a RNA polymerase, and examples thereof may include a T7 promoter, an Sp6 promoter, or a CMV promoter. The vector may include a promoter operable in animal cells, preferably mammalian cells. According to an embodiment, suitable promoters may include promoters derived from mammalian viruses and promoters derived from the genome of mammalian cells, and examples thereof may include a cytomegalovirus (CMV) promoter, a U6 promoter, an H1 promoter, a murine leukemia virus (MLV)-long terminal repeat (LTR) promoter, an adenovirus early promoter, an adenovirus late promoter, a vaccinia virus 7.5K promoter, an SV40 promoter, a tk promoter of HSV, an RSV promoter, an EF1 alpha promoter, a metallothionine promoter, a beta-actin promoter, a promoter of human IL-2 gene, a promoter of human IFN gene, a promoter of human IL-4 gene, a promoter of human lymphotoxin gene, a promoter of human GM-CSF gene, a human phosphoglycerate kinase (PGK) promoter, a mouse phosphoglycerate kinase (PGK) promoter, and a surviving promoter. In an embodiment, the vector may be a plasmid vector including gene segments of cDNA of rotavirus or DNA encoding a capping enzyme. For example, the vector may be a plasmid having the cleavage map of FIG. 2 or 3.

In an embodiment, the reassortant rotavirus may have any one of serotypes G1, G2, G3, G4, G9, and P1, and in the gene segments of rotavirus cDNA in the expression vector for preparing the reassortant rotavirus, a gene segment of VP4 cDNA may have a nucleotide sequence of SEQ ID NO: 5 or 6, and a gene segment of VP7 cDNA may have a nucleotide sequence of SEQ ID NO: 8, 9, 10, 11, 12, or 13. The genome segments of VP4 and VP7 may determine the serotype of the reassortant rotavirus, and through a combination with VP1, VP2, VP3, VP6, NSP1, NSP2, NSP3, NSP4, and NSP5, the reassortant rotavirus having a desired serotype may be prepared.

In an embodiment, in the gene segments of rotavirus cDNA in the expression vector for preparing the reassortant rotavirus, a gene segment of VP1 cDNA may have a nucleotide sequence of SEQ ID NO: 1; a gene segment of VP2 cDNA may have a nucleotide sequence of SEQ ID NO: 2; a gene segment of VP3 cDNA may have a nucleotide sequence of SEQ ID NO: 3 or 4; a gene segment of VP6 cDNA may have a nucleotide sequence of SEQ ID NO: 7; a gene segment of NSP1 cDNA may have a nucleotide sequence of SEQ ID NO: 14; a gene segment of NSP2 cDNA may have a nucleotide sequence of SEQ ID NO: 15; a gene segment of NSP3 cDNA may have a nucleotide sequence of SEQ ID NO: 16; a gene segment of NSP4 cDNA may have a nucleotide sequence of SEQ ID NO: 17; or a gene segment of NSP5 cDNA may have a nucleotide sequence of SEQ ID NO: 18.

In an embodiment, the expression vector library for preparing the reassortant rotavirus may consist of expression vectors each including the following combinations of gene segments of cDNA: (A) VP1 cDNA of SEQ ID NO: 1, VP2 cDNA of SEQ ID NO: 2, VP3 cDNA of SEQ ID NO: 3, VP4 cDNA of SEQ ID NO: 5, VP6 cDNA of SEQ ID NO: 7, VP7 cDNA of SEQ ID NO: 8, NSP1 cDNA of SEQ ID NO: 14, NSP2 cDNA of SEQ ID NO: 15, NSP3 cDNA of SEQ ID NO: 16, NSP4 cDNA of SEQ ID NO: 17, and NSP5 cDNA of SEQ ID NO: 18; (B) VP1 cDNA of SEQ ID NO: 1, VP2 cDNA of SEQ ID NO: 2, VP3 cDNA of SEQ ID NO: 3, VP4 cDNA of SEQ ID NO: 5, VP6 cDNA of SEQ ID NO: 7, VP7 cDNA of SEQ ID NO: 9, NSP1 cDNA of SEQ ID NO: 14, NSP2 cDNA of SEQ ID NO: 15, NSP3 cDNA of SEQ ID NO: 16, NSP4 cDNA of SEQ ID NO: 17, and NSP5 cDNA of SEQ ID NO: 18; (C) VP1 cDNA of SEQ ID NO: 1, VP2 cDNA of SEQ ID NO: 2, VP3 cDNA of SEQ ID NO: 4, VP4 cDNA of SEQ ID NO: 5, VP6 cDNA of SEQ ID NO: 7, VP7 cDNA of SEQ ID NO: 10, NSP1 cDNA of SEQ ID NO: 14, NSP2 cDNA of SEQ ID NO: 15, NSP3 cDNA of SEQ ID NO: 16, NSP4 cDNA of SEQ ID NO: 17, and NSP5 cDNA of SEQ ID NO: 18; (D) VP1 cDNA of SEQ ID NO: 1, VP2 cDNA of SEQ ID NO: 2, VP3 cDNA of SEQ ID NO: 4, VP4 cDNA of SEQ ID NO: 5, VP6 cDNA of SEQ ID NO: 7, VP7 cDNA of SEQ ID NO: 11, NSP1 cDNA of SEQ ID NO: 14, NSP2 cDNA of SEQ ID NO: 15, NSP3 cDNA of SEQ ID NO: 16, NSP4 cDNA of SEQ ID NO: 17, and NSP5 cDNA of SEQ ID NO: 18; (E) VP1 cDNA of SEQ ID NO: 1, VP2 cDNA of SEQ ID NO: 2, VP3 cDNA of SEQ ID NO: 4, VP4 cDNA of SEQ ID NO: 5, VP6 cDNA of SEQ ID NO: 7, VP7 cDNA of SEQ ID NO: 12, NSP1 cDNA of SEQ ID NO: 14, NSP2 cDNA of SEQ ID NO: 15, NSP3 cDNA of SEQ ID NO: 16, NSP4 cDNA of SEQ ID NO: 17, and NSP5 cDNA of SEQ ID NO: 18; and (F) VP1 cDNA of SEQ ID NO: 1, VP2 cDNA of SEQ ID NO: 2, VP3 cDNA of SEQ ID NO: 4, VP4 cDNA of SEQ ID NO: 6, P6 cDNA of SEQ ID NO: 7, VP7 cDNA of SEQ ID NO: 13, NSP1 cDNA of SEQ ID NO: 14, NSP2 cDNA of SEQ ID NO: 15, NSP3 cDNA of SEQ ID NO: 16, NSP4 cDNA of SEQ ID NO: 17, and NSP5 cDNA of SEQ ID NO: 18. The combinations of the aforementioned gene segments of cDNA and the serotypes result in elimination of the pathogenicity of human rotaviruses and addition of genes of bovine rotaviruses harmless to the human body simultaneously, and by including epitope portions (e.g., VP7 and VP4) that can optimally form immunogenicity against human rotaviruses, the immunogenicity of the combinations may be maximized.

In an embodiment, the expression vector library may include an expression cassette that additionally includes a ribozyme-coding sequence flanked by the gene segments of cDNA. The expression vector library for rotavirus may include at least one plasmid selected from the group consisting of: a plasmid consisting of a nucleotide sequence of SEQ ID NO: 19 and including an expression cassette for VP1 gene segments; a plasmid consisting of a nucleotide sequence of SEQ ID NO: 20 and including an expression cassette for VP2 gene segments; a plasmid consisting of a nucleotide sequence of SEQ ID NO: 21 or 22 and including an expression cassette for VP3 gene segments; a plasmid consisting of a nucleotide sequence of SEQ ID NO: 23 or 24 and including an expression cassette for VP4 gene segments; a plasmid consisting of a nucleotide sequence of SEQ ID NO: 25 and including an expression cassette for VP6 gene segments; a plasmid consisting of a nucleotide sequence of SEQ ID NO: 26, 27, 28, 29, 30, or 31 and including an expression cassette for VP7 gene segments; a plasmid consisting of a nucleotide sequence of SEQ ID NO: 32 and including an expression cassette for NSP1 gene segments; a plasmid consisting of a nucleotide sequence of SEQ ID NO: 33 and including an expression cassette for NSP2 gene segments; a plasmid consisting of a nucleotide sequence of SEQ ID NO: 34 and including an expression cassette for NSP3 gene segments; a plasmid consisting of a nucleotide sequence of SEQ ID NO: 35 and including an expression cassette for NSP4 gene segments; and a plasmid consisting of a nucleotide sequence of SEQ ID NO: 36 and including an expression cassette for NSP5 gene segments. In addition, combinations of the expression cassettes may be also prepared correspondingly to the combination of the aforementioned gene segments of cDNA.

In an embodiment, the expression vector library for rotavirus may include at least one plasmid selected from the group consisting of: a plasmid consisting of a nucleotide sequence of SEQ ID NO: 37 and including genome segments of VP1 cDNA; a plasmid consisting of a nucleotide sequence of SEQ ID NO: 38 and including genome segments of VP2 cDNA; a plasmid consisting of a nucleotide sequence of SEQ ID NO: 39 or 40 and including genome segments of VP3 cDNA; a plasmid consisting of a nucleotide sequence of SEQ ID NO: 41 or 42 and including genome segments of VP4 cDNA; a plasmid consisting of a nucleotide sequence of SEQ ID NO: 43 and including genome segments of VP6 cDNA; a plasmid consisting of a nucleotide sequence of SEQ ID NO: 44, 45, 46, 47, 48, or 49 and including genome segments of VP7 cDNA; a plasmid consisting of a nucleotide sequence of SEQ ID NO: 50 and including genome segments of NSP1 cDNA; a plasmid consisting of a nucleotide sequence of SEQ ID NO: 51 and including genome segments of NSP2 cDNA; a plasmid consisting of a nucleotide sequence of SEQ ID NO: 52 and including genome segments of NSP3 cDNA; a plasmid consisting of a nucleotide sequence of SEQ ID NO: 53 and including genome segments of NSP4 cDNA; and a plasmid consisting of a nucleotide sequence of SEQ ID NO: 54 and including genome sequences of NSP5 cDNA. In addition, combinations of the plasmids may be also prepared correspondingly to the combination of the aforementioned gene segments of cDNA.

In an embodiment, the expression vector library for rotavirus may further include: a plasmid consisting of a nucleotide sequence of SEQ ID NO: 37 and including D1R; and a plasmid consisting of a nucleotide sequence of SEQ ID NO: 38 and including D12L.

In an embodiment, in the expression vector library, the molar ratio of the plasmid including gene segments of NSP2 or NSP5 cDNA to the plasmid including gene segments of VP1, VP2, VP3, VP4, VP6, VP7, NSP1, NSP3, or NSP4 cDNA may be 4:1 to 6:1, and for example, the molar ratio between the expression vectors may be 5.0:1 to 5.5:1, 4.0:1 to 5.0:1, 4:1 to 4.5:1, 4.5:1 to 6.0:1, 4.5:1 to 5.5:1, 4.5:1 to 5.0:1, 5.0:1 to 6.0:1, or 5.0:1 to 5.5: 1. When a difference in the molar ratio between the expression vectors occurs beyond the ranges above, the yield of a desired reassortant virus may be significantly reduced or may not be obtained at all, due to the genetic characteristics of the *Reoviridae* virus.

### Advantageous Effects

According to a method for producing a reassortant Reoviridae virus, this method is safer than existing methods using viruses, and without a separate screening process, a reassortant virus of interest can be easily obtained with high efficiency through any combination of vector libraries.

Therefore, the method of producing a reassortant Reoviridae virus according to one aspect and a reassortant Reoviridae virus produced by the method may be used in pathophysiological research on viral infections such as rotavirus infections and in the fields of vaccines for preventing rotavirus injections.

### Description of Drawings

FIG. 1 shows the expression and activity of T7 RNA polymerase in a total of three cell lines (BHK-T7 #3, #6, and #7 cell lines) that stably expresses T7 RNA polymerase, confirmed by fluorescence microscopy, wherein FIG. 1(A) shows a result for the BHK-T7 #3 cell line, FIG. 1(B) shows a result for the BHK-T7 #6 cell line, and FIG. 1(C) shows a result for the BHK-T7 #7 cell line.
FIG. 2 is a cleavage map of a plasmid capable of expressing rotavirus gene segments.
FIG. 3 is a cleavage map of a helper plasmid, wherein FIG. 3(A) is a cleavage map of a pCMVTK-D1R plasmid, and FIG. 3(B) is a cleavage map of a pCMVTK-D12L plasmid.
FIG. 4 is a diagram schematically showing a method of producing reassortant rotavirus by using a cell line into which a T7 RNA polymerase is introduced and an expression vector library for rotavirus, according to an embodiment.
FIG. 5 is a diagram schematically showing a process of producing reassortant rotavirus over time, according to an embodiment.
FIG. 6 is a diagram schematically showing amplification and reinfection processes for reassortant rotavirus over time, according to an embodiment.
FIG. 7 shows a result of confirming occurrence of CPE phenomenon through an electron microscopy, in MA104 cells reinfected with reassortant rotavirus, according to an embodiment.
FIG. 8 shows results of confirming expression of VP6 through a fluorescence microscopy, in MA104 cells reinfected with reassortant rotavirus, according to an embodiment.

### BEST MODE

### Mode for Invention

Hereinafter, preferable Examples are presented to help understanding of the present disclosure. However, Examples below are only presented for easier understanding of the present disclosure, and the contents of the present disclosure are not limited by the following examples.

### Example 1. Preparation of cell line stably expressing T7 RNA polymerase

In this example, genes of T7 RNA polymerase were introduced to BHK21 cell lines, and blasticidin-resistant cell lines were selected therefrom to obtain cell lines stably expressing T7 RNA polymerase. In detail, the synthesized genes of T7 RNA polymerase were inserted upstream of a CMV promoter in an expression vector for the blasticidin-resistant cell genes to prepare an expression vector for the genes of T7 RNA polymerase. Afterwards, the BHK21 cell lines were seeded into a 6-well plate at a concentration of 6.5 × 10⁵ cells/well. Here, as a culture medium, DMEM supplemented with 10 % FBS was used. Afterwards, together with TransIT-LT1 at a concentration of 6 µl/well, the expression vector was added at a concentration of 2 µg/well, and the cells were cultured. Afterwards, the culture medium was replaced with a 10 % DMEM medium supplemented with blasticidin added at a concentration of 10 µg/well, and T7-BHK21 cells survived against the blasticidin resistance were screened. Through the screening process, cell lines in which the genes of a T7 polymerase were inserted into the genome and stably maintained therein were obtained, and through single cell isolation, single clones of the cell lines (BHK-T7 #3, #6, and #7 cell lines) were also obtained.

Meanwhile, to evaluate expression and activity of the T7 RNA polymerase in the obtained cell lines, a reporter plasmid was prepared by synthesizing and inserting a T7 promoter-EMCV IRES-GFP gene into a pUC57 cloning vector, and the reporter plasmid was then transfected into the BHK-T7 #3, #6, or #7 cell line. 2 days after the transfection, the expression level of GFP according to the expression of T7 RNA polymerase was confirmed through a fluorescence microscope.

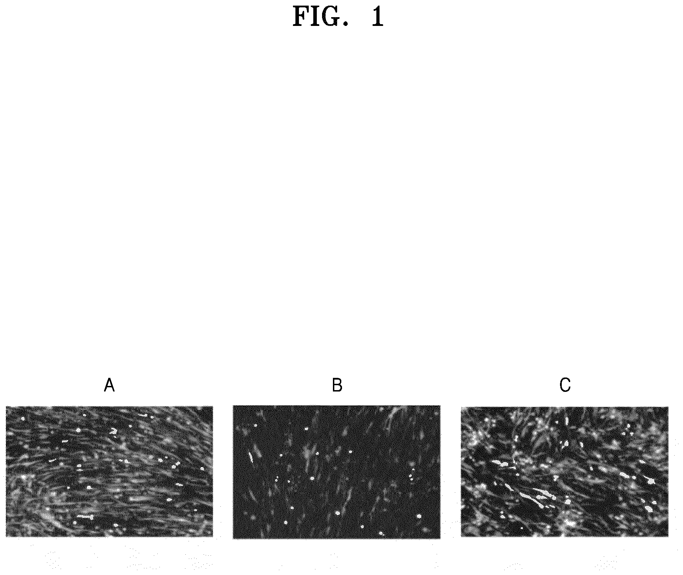

As a result, as shown in FIG. 1, it was confirmed that T7 RNA polymerase expressed at a high level in the BHK-T7 #3, #6, or #7 cell line according to an embodiment.

### Example 2. Preparation of expression vector library for production of reassortant rotavirus

In this example, reassortant rotavirus, in which a total of 11 gene segments of a bovine rotavirus and a human rotavirus were reasserted, was to be produced through a plasmid-based reverse genetics system, wherein the reassortant rotavirus is specifically the one for production of a hexavalent vaccine strain to prevent infection with a human rotavirus with a G1, G2, G3, G4, G9, or P1 serotype. For this purpose, in this example, a plasmid containing gene segments of cDNA of rotavirus and a helper plasmid expressing a capping enzyme of vaccinia virus were prepared.

### 2-1. Preparation of plasmid containing gene segments of rotavirus

Table 1 shows the genetic information of each gene segment of rotavirus for each serotype and a combination of the genetic information. In Table 1, the genetic information for each gene segment is indicated by the GenBank number, the gene derived from bovine rotavirus WC3 is indicated by (B), and the gene derived from human rotavirus is indicated by (H).

**[Table 1]**

| **Serot ype** | **VP1** | **VP2** | **VP3** | **VP4** | **VP6** | **VP7** | **NSP 1** | **NSP 2** | **NSP 3** | **NSP 4** | **NSP 5** |
|---|---|---|---|---|---|---|---|---|---|---|---|
| **G1** | GU5 6505 2 (B) | GU5 6505 3 (B) | GU5 6505 4 (H) | GU5 6506 6 (B) | GU5 6505 6 (B) | GU5 6505 7 (H) | GU5 6505 8 (B) | GU5 6505 9 (B) | GU5 6507 1 (B) | GU5 6506 1 (B) | GU5 6506 2 (B) |
| **G2** | GU5 6505 2 (B) | GU5 6505 3 (B) | GU5 6505 4 (H) | GU5 6506 6 (B) | GU5 6505 6 (B) | GU5 6506 8 (H) | GU5 6505 8 (B) | GU5 6505 9 (B) | GU5 6507 1 (B) | GU5 6506 1 (B) | GU5 6506 2 (B) |
| **G3** | GU5 6505 2 (B) | GU5 6505 3 (B) | GU5 6507 6 (B) | GU5 6506 6 (B) | GU5 6505 6 (B) | GU5 6507 9 (H) | GU5 6505 8 (B) | GU5 6505 9 (B) | GU5 6507 1 (B) | GU5 6506 1 (B) | GU5 6506 2 (B) |
| **G4** | GU5 6505 2 (B) | GU5 6505 3 (B) | GU5 6507 6 (B) | GU5 6506 6 (B) | GU5 6505 6 (B) | GU5 6509 0 (H) | GU5 6505 8 (B) | GU5 6505 9 (B) | GU5 6507 1 (B) | GU5 6506 1 (B) | GU5 6506 2 (B) |
| **G9** | GU5 6505 2 (B) | GU5 6505 3 (B) | GU5 6507 6 (B) | GU5 6506 6 (B) | GU5 6505 6 (B) | AB1 8096 9 (H) | GU5 6505 8 (B) | GU5 6505 9 (B) | GU5 6507 1 (B) | GU5 6506 1 (B) | GU5 6506 2 (B) |
| **P1** | GU5 6505 2 (B) | GU5 6505 3 (B) | GU5 6507 6 (B) | GU5 6504 4 (H) | GU5 6505 6 (B) | GU5 6504 6 (B) | GU5 6505 8 (B) | GU5 6505 9 (B) | GU5 6507 1 (B) | GU5 6506 1 (B) | GU5 6506 2 (B) |

For the expression of the gene segments of rotavirus in Table 1, as shown in FIG. 2, an expression cassette in which cDNA of the gene segments is located immediately upstream of a T7 promoter sequence and the cDNA is followed by an antigenomic HDV ribozyme sequence and a T7 terminator sequence was designed. Then, the expression cassette was inserted into a pUC57 cloning vector to prepare a plasmid containing the cDNA of the gene segments of rotavirus.

Meanwhile, Table 2 shows the information on the plasmid containing the cDNA of the gene segments of rotavirus prepared in one example.

**[Table 2]**

| **Plasmid name** | **Rota cDNA** | **Rota gene** | **Vector** | **Cloning sites** | **Length (bp)** | **SEQ ID NO:** |
|---|---|---|---|---|---|---|
| pTK-VP1B | GU565052 | VP1 (B) | pUC57 | KpnI/SalI | 6203 | 19 |
| pTK-VP2B | GU565053 | VP2 (B) | pUC57 | KpnI/SalI | 5588 | 20 |
| pTK-VP3H | GU565054 | VP3 (H) | pUC57 | KpnI/SalI | 5492 | 21 |
| pTK-VP3B | GU565076 | VP3 (B) | pUC57 | KpnI/SalI | 5492 | 22 |
| pTK-VP4B | GU565066 | VP4 (B) | pUC57 | EcoRI/KpnI | 5291 | 23 |
| pTK-VP4P1 | GU565044 | VP4 (H; P1) | pUC57 | KpnI/SalI | 5260 | 24 |
| pTK-VP6B | GU565056 | VP6 (B) | pUC57 | KpnI/SalI | 4257 | 25 |
| pTK-VP7G1 | GU565057 | VP7 (H; G1) | pCC1 | KpnI/SalI | 9352 | 26 |
| pTK-VP7G2 | GU565068 | VP7 (H; G2) | pUC57 | KpnI/SalI | 3963 | 27 |
| pTK-VP7G3 | GU565079 | VP7 (H; G3) | pUC57 | KpnI/SalI | 3963 | 28 |
| pTK-VP7G4 | GU565090 | VP7 (H; G4) | pUC57 | KpnI/SalI | 3963 | 29 |
| pTK-VP7G9 | AB180969 | VP7 (H; G9) | pUC57 | KpnI/SalI | 3962 | 30 |
| pTK-VP7B | GU565046 | VP7 (B) | pUC57 | KpnI/SalI | 3963 | 31 |
| pTK-NSP1B | GU565058 | NSP1 (B) | pUC57 | KpnI/SalI | 4479 | 32 |
| pTK-NSP2B | GU565059 | NSP2 (B) | pUC57 | KpnI/SalI | 3960 | 33 |
| pTK-NSP3B | GU565071 | NSP3 (B) | pUC57 | EcoRI/SalI | 3963 | 34 |
| pTK-NSP4B | GU565061 | NSP4 (B) | pUC57 | EcoRI/KpnI | 3680 | 35 |
| pTK-NSP5B | GU565062 | NSP5 (B) | pUC57 | KpnI/SalI | 3568 | 36 |

### 2-2. Preparation of helper plasmid

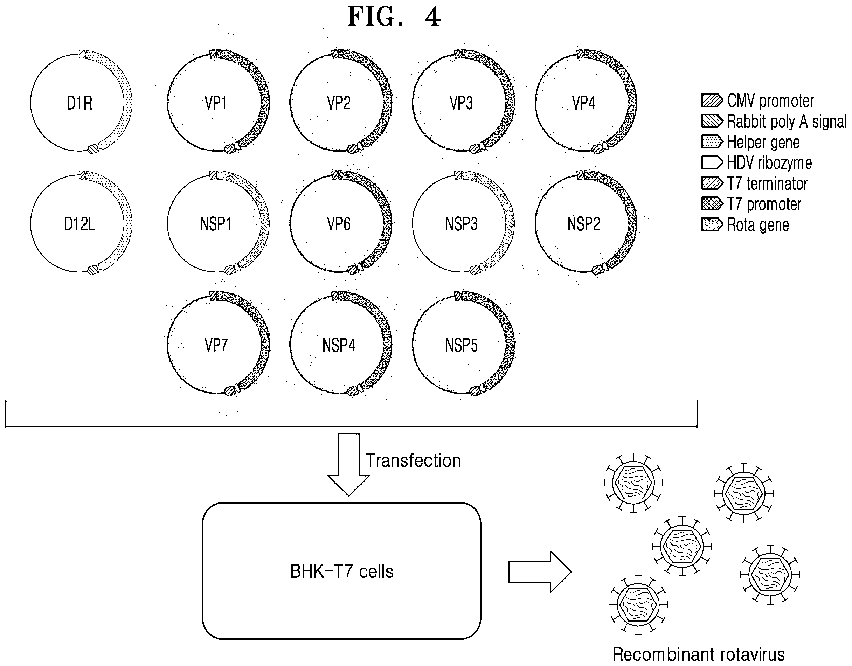

To prepare a helper plasmid, an expression vector (pCMVTK) to which a CMV immediately early promoter, a multi cloning site (MCS), and a rabbit globin poly A signal sequence were synthesized and inserted was designed. Then, as shown in FIG. 4, two types of helper plasmids, pCMVTK-D1R and pCMVTK-D12L, were prepared by inserting D1R genes or D12L genes into the pCMVTK vector.

### Example 3. Preparation of reassortant rotavirus

In this example, as shown in FIG. 4, rotavirus in which a total of 11 gene segments were reassorted was to be prepared by transfecting the cell lines stably expressing T7 RNA polymerase of Example 1 into a DNA mixture selected from the expression vector library of Example 2.

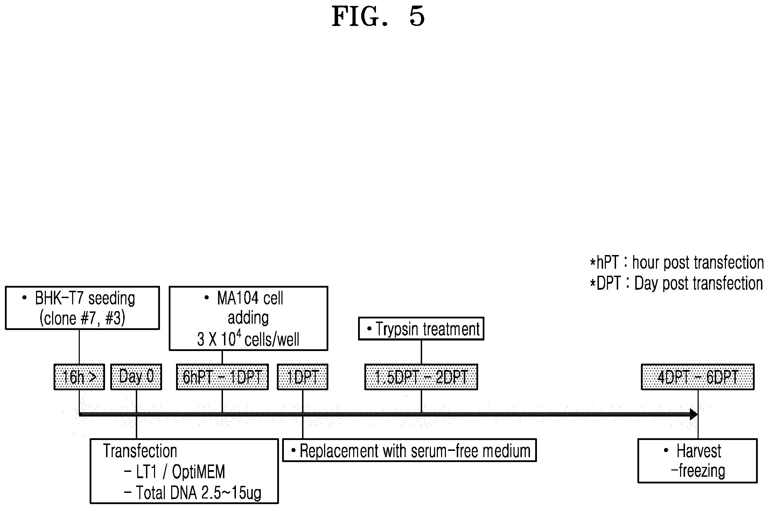

FIG. 5 is a diagram schematically showing a process of producing reassortant rotavirus over time, according to an embodiment. In detail, the BHK-T7 cell line was seeded into a 6-well plate about 16 hours prior to transfection such that the BHK-T7 cell line with a confluency of 80 % to 90 % at the time of transfection (Day 0) was prepared in a monolayer. Here, as a medium, DMEM supplemented with 5 % to 10 % FBS was used. Meanwhile, MA104 cells were seeded into a 6-well plate such that, for use in a subsequent process of adding the MA104 cells, the MA104 cells with a confluency of 80 % to 90 % were prepared. Here, as a medium, DMEM supplemented with 5 % FBS was used.

About at least 16 hours after the seeding of the BHK-T7 cells, transfection with the DNA mixture selected from the vector library was performed when the cells reached a confluency of 80 % to 90 %. The DNA mixture was composed of 11 plasmids expressing each gene segment of rotavirus and 2 helper plasmids, and the composition of the DNA mixture used for the production of reassortant rotavirus with a G1, G2, G3, G4, G9, or P1 serotype are shown in Tables 3 to 8. The level of Opti-MEM (Gibco) was adjusted in proportion to the total DNA level, i.e., the plasmid level, and accordingly, 250 ul of Opti-MEM was added per 2.5 ug of DNA. Afterwards, as a transfection reagent, a TransIT-LT1 transfection reagent (mirus Bio) was added at a level of 3 ul per 1 ug of the total DNA, mixed gently and thoroughly by pipetting, and incubated the mixture at room temperature for 15 minutes to 30 minutes.

Tables 3 to 8 provide the information on the combinations of plasmids and contents of plasmids for the production of reassortant rotavirus with a G1, G2, G3, G4, G9, or P1 serotype.

**[Table 3]**

| **G1** | **Plasmid name** | **Rota gene** | **Length (bp)** | **Molar ratio** | **Range of Use of DNA (ug)** | |
|---|---|---|---|---|---|---|
| **DNA mixture** | pTK-VP1B | VP1 (B) | 6203 | 1 | 0.16 | 0.96 |
| | pTK-VP2B | VP2 (B) | 5588 | 1 | 0.14 | 0.86 |
| | pTK-VP3H | VP3 (H) | 5492 | 1 | 0.14 | 0.85 |
| | pTK-VP4B | VP4 (B) | 5291 | 1 | 0.14 | 0.82 |
| | pTK-VP6B | VP6 (B) | 4257 | 1 | 0.11 | 0.66 |
| | pTK-VP7G1 | VP7 (H; G1) | 9352 | 1 | 0.24 | 1.45 |
| | pTK-NSP1B | NSP1 (B) | 4479 | 1 | 0.12 | 0.69 |
| | pTK-NSP2B | NSP2 (B) | 3960 | 5 | 0.51 | 3.06 |
| | pTK-NSP3B | NSP3 (B) | 3963 | 1 | 0.10 | 0.61 |
| | pTK-NSP4B | NSP4 (B) | 3680 | 1 | 0.09 | 0.57 |
| | pTK-NSP5B | NSP5 (B) | 3568 | 5 | 0.46 | 2.76 |
| | pCMVTK-D1R | helper | 6364 | 1 | 0.16 | 0.98 |
| | pCMVTK-D12L | helper | 4693 | 1 | 0.12 | 0.73 |
| **Total DNA (ug)** | | | | | 2.5 | 15 |
| **OptiMEM** | | | | | 250 | 1500 |
| **LT1** | | | | | 7.5 | 45 |

**[Table 4]**

| **G2** | **Plasmid name** | **Rota gene** | **Length (bp)** | **Molar ratio** | **Range of Use of DNA (ug)** | |
|---|---|---|---|---|---|---|
| **DNA mixture** | pTK-VP1B | VP1 (B) | 6203 | 1 | 0.17 | 1.02 |
| | pTK-VP2B | VP2 (B) | 5588 | 1 | 0.15 | 0.91 |
| | pTK-VP3H | VP3 (H) | 5492 | 1 | 0.15 | 0.90 |
| | pTK-VP4B | VP4 (B) | 5291 | 1 | 0.14 | 0.87 |
| | pTK-VP6B | VP6 (B) | 4257 | 1 | 0.12 | 0.70 |
| | pTK-VP7G2 | VP7 (H; G2) | 3963 | 1 | 0.11 | 0.65 |
| | pTK-NSP1B | NSP1 (B) | 4479 | 1 | 0.12 | 0.73 |
| | pTK-NSP2B | NSP2 (B) | 3960 | 5 | 0.54 | 3.24 |
| | pTK-NSP3B | NSP3 (B) | 3963 | 1 | 0.11 | 0.65 |
| | pTK-NSP4B | NSP4 (B) | 3680 | 1 | 0.10 | 0.60 |
| | pTK-NSP5B | NSP5 (B) | 3568 | 5 | 0.49 | 2.92 |
| | pCMVTK-D1R | helper | 6364 | 1 | 0.17 | 1.04 |
| | pCMVTK-D12L | helper | 4693 | 1 | 0.13 | 0.77 |
| **Total DNA (ug)** | | | | | 2.5 | 15 |
| **OptiMEM** | | | | | 250 | 1500 |
| **LT1** | | | | | 7.5 | 45 |

**[Table 5]**

| | | | | | | |
|---|---|---|---|---|---|---|
| **G3** | **Plasmid name** | **Rota gene** | **Length (bp)** | **Molar ratio** | **Range of Use of DNA (ug)** | |
| **DNA mixture** | pTK-VP1B | VP1 (B) | 6203 | 1 | 0.17 | 1.02 |
| | pTK-VP2B | VP2 (B) | 5588 | 1 | 0.15 | 0.91 |
| | pTK-VP3B | VP3 (B) | 5492 | 1 | 0.15 | 0.90 |
| | pTK-VP4B | VP4 (B) | 5291 | 1 | 0.14 | 0.87 |
| | pTK-VP6B | VP6 (B) | 4257 | 1 | 0.12 | 0.70 |
| | pTK-VP7G3 | VP7 (H; G3) | 3963 | 1 | 0.11 | 0.65 |
| | pTK-NSP1B | NSP1 (B) | 4479 | 1 | 0.12 | 0.73 |
| | pTK-NSP2B | NSP2 (B) | 3960 | 5 | 0.54 | 3.24 |
| | pTK-NSP3B | NSP3 (B) | 3963 | 1 | 0.11 | 0.65 |
| | pTK-NSP4B | NSP4 (B) | 3680 | 1 | 0.10 | 0.60 |
| | pTK-NSP5B | NSP5 (B) | 3568 | 5 | 0.49 | 2.92 |
| | pCMVTK-D1R | helper | 6364 | 1 | 0.17 | 1.04 |
| | pCMVTK-D12L | helper | 4693 | 1 | 0.13 | 0.77 |
| **Total DNA (ug)** | | | | | 2.5 | 15 |
| **OptiMEM** | | | | | 250 | 1500 |
| **LT1** | | | | | 7.5 | 45 |

**[Table 6]**

| **G4** | **Plasmid name** | **Rota gene** | **Length (bp)** | **Molar ratio** | **Range of Use of DNA (ug)** | |
|---|---|---|---|---|---|---|
| **DNA mixture** | pTK-VP1B | VP1 (B) | 6203 | 1 | 0.17 | 1.02 |
| | pTK-VP2B | VP2 (B) | 5588 | 1 | 0.15 | 0.91 |
| | pTK-VP3B | VP3 (B) | 5492 | 1 | 0.15 | 0.90 |
| | pTK-VP4B | VP4 (B) | 5291 | 1 | 0.14 | 0.87 |
| | pTK-VP6B | VP6 (B) | 4257 | 1 | 0.12 | 0.70 |
| | pTK-VP7G4 | VP7 (H; G4) | 3963 | 1 | 0.11 | 0.65 |
| | pTK-NSP1B | NSP1 (B) | 4479 | 1 | 0.12 | 0.73 |
| | pTK-NSP2B | NSP2 (B) | 3960 | 5 | 0.54 | 3.24 |
| | pTK-NSP3B | NSP3 (B) | 3963 | 1 | 0.11 | 0.65 |
| | pTK-NSP4B | NSP4 (B) | 3680 | 1 | 0.10 | 0.60 |
| | pTK-NSP5B | NSP5 (B) | 3568 | 5 | 0.49 | 2.92 |
| | pCMVTK-D1R | helper | 6364 | 1 | 0.17 | 1.04 |
| | pCMVTK-D12L | helper | 4693 | 1 | 0.13 | 0.77 |
| **Total DNA (ug)** | | | | | 2.5 | 15 |
| **OptiMEM** | | | | | 250 | 1500 |
| **LT1** | | | | | 7.5 | 45 |

**[Table 7]**

| **G9** | **Plasmid name** | **Rota gene** | **Length (bp)** | **Molar ratio** | **Range of Use of DNA (ug)** | |
|---|---|---|---|---|---|---|
| **DNA mixture** | pTK-VP1B | VP1 (B) | 6203 | 1 | 0.17 | 1.02 |
| | pTK-VP2B | VP2 (B) | 5588 | 1 | 0.15 | 0.91 |
| | pTK-VP3B | VP3 (B) | 5492 | 1 | 0.15 | 0.90 |
| | pTK-VP4B | VP4 (B) | 5291 | 1 | 0.14 | 0.87 |
| | pTK-VP6B | VP6 (B) | 4257 | 1 | 0.12 | 0.70 |
| | pTK-VP7G9 | VP7 (H; G9) | 3962 | 1 | 0.11 | 0.65 |
| | pTK-NSP1B | NSP1 (B) | 4479 | 1 | 0.12 | 0.73 |
| | pTK-NSP2B | NSP2 (B) | 3960 | 5 | 0.54 | 3.24 |
| | pTK-NSP3B | NSP3 (B) | 3963 | 1 | 0.11 | 0.65 |
| | pTK-NSP4B | NSP4 (B) | 3680 | 1 | 0.10 | 0.60 |
| | pTK-NSP5B | NSP5 (B) | 3568 | 5 | 0.49 | 2.92 |
| | pCMVTK-D1R | helper | 6364 | 1 | 0.17 | 1.04 |
| | pCMVTK-D12L | helper | 4693 | 1 | 0.13 | 0.77 |
| **Total DNA (ug)** | | | | | 2.5 | 15 |
| **OptiMEM** | | | | | 250 | 1500 |
| **LT1** | | | | | 7.5 | 45 |

**[Table 8]**

| **P1** | **Plasmid name** | **Rota gene** | **Length (bp)** | **Molar ratio** | **Range of Use of DNA (ug)** | |
|---|---|---|---|---|---|---|
| **DNA mixture** | pTK-VP1B | VP1 (B) | 6203 | 1 | 0.17 | 1.02 |
| | pTK-VP2B | VP2 (B) | 5588 | 1 | 0.15 | 0.92 |
| | pTK-VP3B | VP3 (B) | 5492 | 1 | 0.15 | 0.90 |
| | pTK-VP4P1 | VP4 (H; P1) | 5260 | 1 | 0.14 | 0.86 |
| | pTK-VP6B | VP6 (B) | 4257 | 1 | 0.12 | 0.70 |
| | pTK-VP7B | VP7 (B) | 3963 | 1 | 0.11 | 0.65 |
| | pTK-NSP1B | NSP1 (B) | 4479 | 1 | 0.12 | 0.73 |
| | pTK-NSP2B | NSP2 (B) | 3960 | 5 | 0.54 | 3.24 |
| | pTK-NSP3B | NSP3 (B) | 3963 | 1 | 0.11 | 0.65 |
| | pTK-NSP4B | NSP4 (B) | 3680 | 1 | 0.10 | 0.60 |
| | pTK-NSP5B | NSP5 (B) | 3568 | 5 | 0.49 | 2.92 |
| | pCMVTK-D1R | helper | 6364 | 1 | 0.17 | 1.04 |
| | pCMVTK-D12L | helper | 4693 | 1 | 0.13 | 0.77 |
| **Total DNA (ug)** | | | | | 2.5 | 15 |
| **OptiMEM** | | | | | 250 | 1500 |
| **LT1** | | | | | 7.5 | 45 |

Meanwhile, during the incubation, the medium of the BHK-T7 cell line was removed and an appropriate amount of a fresh growth medium (supplemented with 2.5 ml/ml of the DNA mixture) that has been pre-heated at 37°C was added. After completion of the incubation, the DNA mixture was evenly added to the medium drop by drop, and the cell line was cultured in a CO₂ incubator at 37 °C. Afterwards, the MA104 cells spread in a monolayer with a confluency of 80 % to 90 % were treated with trypsin and separated from the 6-well plate. Then, the MA104 cells were sedimented at 500 xg for 5 minutes, and the resulting supernatant was removed. After 3 ml of GMEM medium supplemented with 5 % FBS was added to the cell pellets and the cells were resuspended, the number of cells was calculated accordingly. Afterwards, the resuspended MA104 cells were added to the medium containing the transfected BHK-T7 cell line, at a concentration of 3×10⁴ cells/well.

About 22 hours to 24 hours after the transfection, the existing medium was removed, washed once or twice with 2.5 ml of serum-free DMEM, and then replaced with serum-free DMEM. About 36 hours to 48 hours after the transfection, trypsin (Sigma cat# T0303) was treated to have a concentration of 0.3 ug/ml to induce conditions favorable for reinfection with a small amount of the resulting reassortant virus. Afterwards, 4 days to 6 days after the transfection, while observing apoptosis of the cells, the plate containing the transfected cells was frozen and stored at -80 °C.

### Example 4. Identification of reassortant rotavirus

In this example, the reassortant rotavirus produced through the processes of Example 3 was to be identified. For this purpose, after the reassortant rotavirus was amplified by infecting the MA104 cells again, the cytopathic effect (CPE) on the cells was confirmed and the expression level of VP6 utilized in the detection of rotavirus infection due to high antigenicity was evaluated.

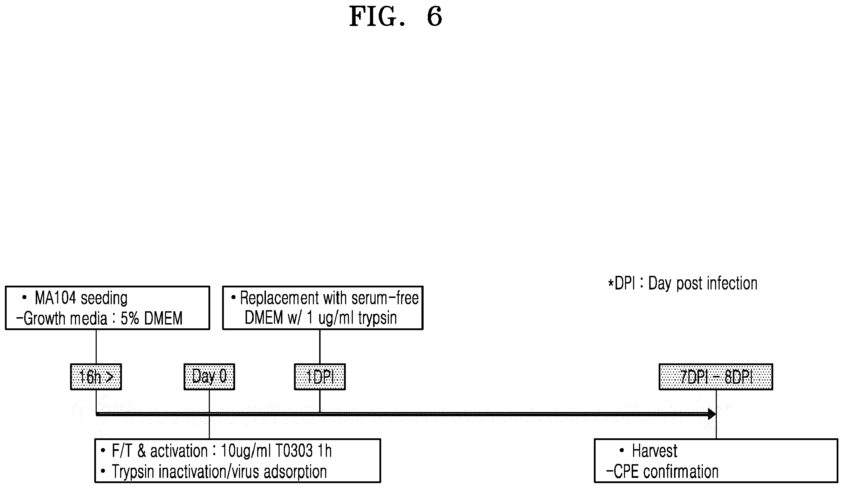

FIG. 6 is a diagram schematically showing the amplification and reinfection processes for the reassortant rotavirus over time, according to an embodiment. In detail, MA104 cells were seeded into a 6-well plate about 16 hours prior to infection such that the MA104 cell with a confluency of 80 % to 90 % at the time of infection (Day 0) were prepared. Here, as a culture medium, DMEM was used. Meanwhile, the plate containing the transfected cells that have been frozen and stored at -80 °C in Example 3 was thawed at room temperature, and a process of re-freezing was repeated twice. Afterwards, the wells containing the transfected cells were treated with trypsin at a concentration of 10 ug/ml and incubated at 37 °C for 1 hour. An infection solution was prepared by adding an equal amount of DMEM supplemented with 10 % FBS to inactivate trypsin. Afterwards, the culture medium of the MA104 cells was removed, and 2.5 ml to 5 ml of the infection solution was added thereto. Then, the resulting cells were cultured in a CO₂ incubator at 37 °C. After 7 days to 8 days, the CPE of the cultured cells was confirmed, and the expression level of GFP according to the expression of VP6 was confirmed through a fluorescence microscope.

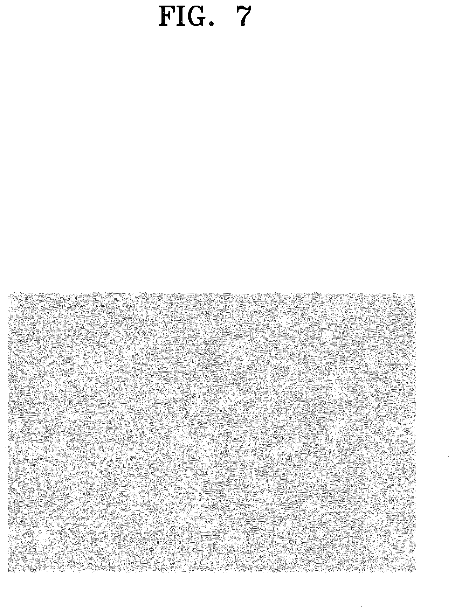

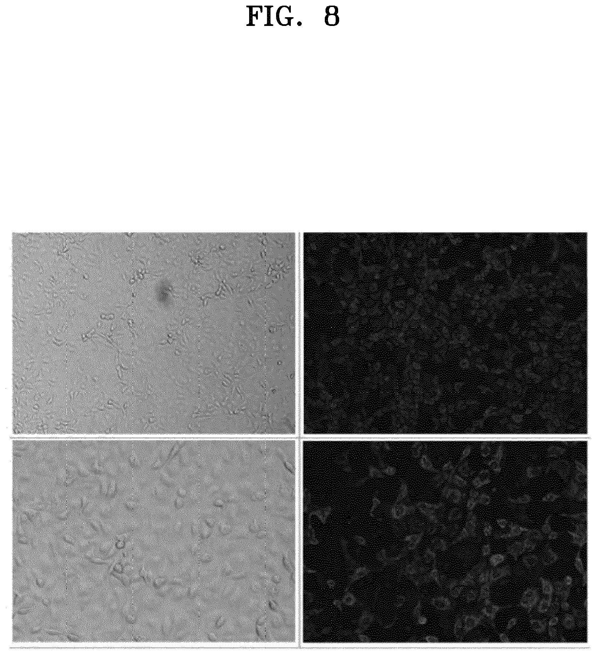

As a result, as shown in FIGS. 7 and 8, it was confirmed that the MA104 cells reinfected with the reassortant rotavirus showed a significant CPE phenomenon, and that VP6 was also expressed at a high level.

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A method for producing a reassortant *Reoviridae* virus, the method comprising:
transfecting a first cell line, into which an RNA polymerase gene is introduced, into an expression vector library comprising a foreign gene;
culturing a first culture by culturing, in a growth medium, the first cell line undergoing the transfection;
culturing a second culture by adding a second cell line to the first culture that has been cultured; and
culturing a third culture by adding trypsin to the second culture that has been cultured,
wherein the expression vector library comprises a gene segment of cDNA of the *Reoviridae* virus and a promoter capable of binding to the RNA polymerase.

2. The method of claim 1, wherein the *Reoviridae* virus is one selected from the group consisting of the genera *Rotavirus*, *Cardoreovirus*, *Mimoreovirus*, *Orbivirus*, *Phytoreovirus, Seadornavirus, Aquareovirus*, *Coltivirus*, *Cypovirus*, *Dinovernavirus*, *Fijivirus*, *Idnoreovirus*, *Mycoreovirus*, *Orthoreovirus*, and *Oryzavirus.*

3. The method of claim 1, wherein the RNA polymerase is a T7 RNA polymerase, a T3 RNA polymerase, an SP6 RNA polymerase, or a mitochondrial polymerase (POLRMT).

4. The method of claim 1, wherein the first cell line is BHK21 cell line, HEK293 cell line, HeLa cell line, CHO cell line, LNCaP cell line, A549 cell line, or hepG2 cell line.

5. The method of claim 1, wherein the second cell line is MA104 cell line, Vero cell line, HEK cell line, human intestinal epithelial cells, HT-29 cell line, Caco2 cell line, LLC-MK2 cell line, FRhL-2 cell line, CV-1 cell line, COS-7 cell line, BSC-1 cell line, or BGM cell line.

6. The method of claim 1, wherein, in the culturing of the second culture, the second cell line is added to the first culture 6 hours to 12 hours after the transfection.

7. The method of claim 1, wherein, in the culturing of the third culture, the trypsin is added to the second culture 36 hours to 48 hours after the transfection.

8. The method of claim 1, wherein the trypsin is added at a concentration of 0.1 ug/ml to 1 ug/ml.

9. The method of claim 1, wherein the promoter is a T7 promoter, an Sp6 promoter, or a CMV promoter.

10. The method of claim 1, wherein a total DNA weight in the expression vector library is 1 ug to 20 ug per 1×10⁴ cells to 1×10⁶ cells.

11. The method of claim 1, wherein the expression vector library further comprises an expression vector for D1R or D12L encoding a capping enzyme.

12. The method of claim 1, wherein, when the growth medium is a medium supplemented with fetal bovine serum, the method further comprises replacing the growth medium with a culture medium not supplemented with fetal bovine serum, prior to the culturing of the third culture.

13. The method of claim 1, wherein the growth medium is Dulbeco's modified Eagle's medium (DMEM), Eagle's minimum essential medium (EMEM) or Glasgow minimum essential medium (GMEM).

14. The method of claim 12, wherein the replacing of the growth medium with the culture medium not supplemented with fetal bovine serum is performed 20 hours after the transfection.

15. A reassortant *Reoviridae* virus produced by the method of any one of claims 1 to 14.

16. An expression vector library for producing reassortant rotavirus, the expression vector library comprising a T7 promoter capable of binding to a T7 RNA polymerase and a plurality of expression vectors each comprising a gene segment of rotavirus cDNA operably linked to the T7 promoter,
wherein the plurality of expression vectors comprise a gene segment of cDNA of one of VP1, VP2, VP3, VP4, VP6, VP7, NSP1, NSP2, NSP3, NSP4, and NSP5.

17. The expression vector library of claim 16, wherein the reassortant rotavirus has one of serotypes G1, G2, G3, G4, G9, and P1, and
in the gene segment of rotavirus cDNA, a gene segment of VP4 cDNA has a nucleotide sequence of SEQ ID NO: 5 or 6, and a gene segment of VP7 cDNA has a nucleotide sequence of SEQ ID NO: 8, 9, 10, 11, 12, or 13.

18. The expression vector library of claim 17, wherein, in the gene segment of rotavirus cDNA, a gene segment of VP1 cDNA has a nucleotide sequence of SEQ ID NO: 1;
a gene segment of VP2 cDNA has a nucleotide sequence of SEQ ID NO: 2;
a gene segment of VP3 cDNA has a nucleotide sequence of SEQ ID NO: 3 or 4;
a gene segment of VP6 cDNA has a nucleotide sequence of SEQ ID NO: 7;
a gene segment of NSP1 cDNA has a nucleotide sequence of SEQ ID NO: 14;
a gene segment of NSP2 cDNA has a nucleotide sequence of SEQ ID NO: 15;
a gene segment of NSP3 cDNA has a nucleotide sequence of SEQ ID NO: 16;
a gene segment of NSP4 cDNA has a nucleotide sequence of SEQ ID NO: 17; and
a gene segment of NSP5 cDNA has a nucleotide sequence of SEQ ID NO: 18.

19. The expression vector library of claim 16, comprising an expression cassette further comprising a ribozyme-coding sequence flanked by the gene segment cDNA.

20. The expression vector library of claim 19, wherein the plurality of expression vectors are each a plasmid.

21. The expression vector library of claim 19, comprising at least one plasmid selected from the group consisting of:
a plasmid consisting of a nucleotide sequence of SEQ ID NO: 20 and comprising an expression cassette for a gene segment of VP2;
a plasmid consisting of a nucleotide sequence of SEQ ID NO: 21 or 22 and comprising an expression cassette for a gene segment of VP3;
a plasmid consisting of a nucleotide sequence of SEQ ID NO: 23 or 24 and comprising an expression cassette for a gene segment of VP4;
a plasmid consisting of a nucleotide sequence of SEQ ID NO: 25 and comprising an expression cassette for a gene segment of VP6;
a plasmid consisting of a nucleotide sequence of SEQ ID NO: 26, 27, 28, 29, 30, or 31 and comprising an expression cassette for a gene segment of VP7;
a plasmid consisting of a nucleotide sequence of SEQ ID NO: 32 and comprising an expression cassette for a gene segment of NSP1;
a plasmid consisting of a nucleotide sequence of SEQ ID NO: 33 and comprising an expression cassette for a gene segment of NSP2;
a plasmid consisting of a nucleotide sequence of SEQ ID NO: 34 and comprising an expression cassette for a gene segment of NSP3;
a plasmid consisting of a nucleotide sequence of SEQ ID NO: 35 and comprising an expression cassette for a gene segment of NSP4; and
a plasmid consisting of a nucleotide sequence of SEQ ID NO: 36 and comprising an expression cassette for a gene segment of NSP5.

22. The expression vector library of claim 21, further comprising: a plasmid for expression of D1R, consisting of a nucleotide sequence of SEQ ID NO: 55; and
a plasmid for expression of D12L, consisting of a nucleotide sequence of SEQ ID NO: 56.

23. The expression vector library of claim 21, wherein a total DNA weight in the expression vector library for rotavirus is 1 ug to 20 ug per 1×10⁴ cells to 1×10⁶ cells.

24. The expression vector library of claim 21, wherein a molar ratio of the plasmid comprising the gene segment of NSP2 or NSP5 cDNA to the plasmid comprising the gene segment of VP1, VP2, VP3, VP4, VP6, VP7, NSP1, NSP3, or NSP4 cDNA is 4:1 to 6:1.
